# EUROPEAN PATENT SPECIFICATION

(11) **EP 2 089 399 B1**
(45) Date of publication and mention of the grant of the patent: **08.08.2012**
(21) Application number: 07857204.7
(22) Date of filing: 09.11.2007
(51) Int. Cl.: C07F 7/00, C07F 11/00, C07C 251/16, C07D 213/53, C07D 215/12, C08F 4/70, C08F 10/00, C08F 4/622

(54) **POLYMERISATION OF ETHYLENE AND ALPHA OLEFINS WITH SINGLE SITE CATALYSTS HAVING AN ANIONIC SCORPION-LIKE LIGAND**
POLYMERISIERUNG VON ETHLYEN UND ALPHA-OLEFINEN MIT SINGLE-SITE-KATALYSATOREN MIT ANIONISCHEM SKORPIONARTIGEM LIGAND
POLYMÉRISATION DE L'ÉTHYLÈNE ET DES ALPHA-OLÉFINES PAR DES CATALYSEURS À SITES INDIVIDUELS AYANT UN LIGANT DE TYPE SCORPION ANIONIQUE

(30) Priority: 24.11.2006 EP 06124706
(43) Date of publication of application: 19.08.2009
(73) Proprietor: Total Petrochemicals Research Feluy, 7181 Seneffe (Feluy) (BE)
(72) Inventor: MICHAUD, Guillaume, 59000 Lille (FR); HILLAIRET, Caroline, 7060 Soignies (BE); SIROL, Sabine, 1440 Wauthier-Braine (BE)
(74) Representative: Leyder, Francis
(86) International application number: PCT/EP2007/062111
(87) International publication number: WO 2008/061900

(56) References cited:
- EP-A- 1 754 723
- WO-A-01/34665

## Description

The present invention related to the field of single site catalyst systems having anionic scorpion-like three dimensional structure that are suitable for oligomerising or polymerising ethylene and alpha-olefins.

There exists a multitude of catalyst systems available for polymerising or oligomerising ethylene and alpha-olefins, but there is a growing need for finding new systems capable to tailor polymers with very specific properties. More and more post-metallocene catalyst components based on early or late transition metals from Groups 3 to 10 of the Periodic Table have recently been investigated such as for example those disclosed in Gibson and al. review (Gibson, V.C.; Spitzmesser, S.K., Chem. Rev. 2003, 103, p. 283). But there is still a need to improve either the specificities or the performances of these systems.

Prior art document WO01/34665 discloses a chain transfer agent for olefin polymerisation. The olefin polymerisation is carried out in the presence of an active polymerisation catalyst comprising a complex of a neutral didentate ligand of a metal selected from the group consisting of nickel, iron and cobalt and of an effective amount of a chain transfer agent.

It is an aim of the present invention to provide a new single site catalyst component based on beta-diimine ligands with a chelating pendant arm.

It is also an aim of the present invention to provide single site catalyst components having a scorpion-like spatial organisation.

It is another aim of the present invention to provide active catalyst systems based on these catalyst components.

It is a further aim of the present invention to provide a process for polymerising or for oligomerising ethylene and alpha-olefins with these new catalyst systems. It is yet a further aim of the present invention to provide a polyethylene by polymerising ethylene with these new catalyst systems.

Accordingly, the present invention discloses a deprotonated ligand of general formula I, 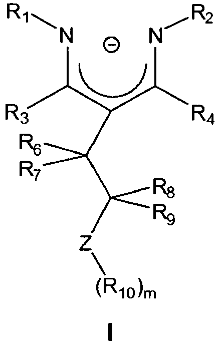 wherein R1, R2, R3, R4, R6, R7, R8, R9 and R10 Z, m have values as indicated in the claims.

By inert functional group, is meant a group, other than hydrocarbyl or substituted hydrocarbyl, that is inert under the complexation conditions to which the compound containing said group is subjected. They can be selected for example from halo, ester, ether, amino, imino, nitro, cyano, carboxyl, phosphate, phosphonite, phosphine, phosphinite, thioether and amide. Preferably, they are selected from halo, such as chloro, bromo, fluoro and iodo, or ether of formula - OR* wherein R* is unsubstituted or substituted hydrocarbyl. After metallation of the ligand, an inert functional group must not coordinate to the metal more strongly than the groups organised to coordinate to the metal and thereby displace the desired coordinating group.

Ligand I results from the reaction between a beta-diimine 11 and a compound of formula III wherein X is a leaving group, preferably halogen for example Br, 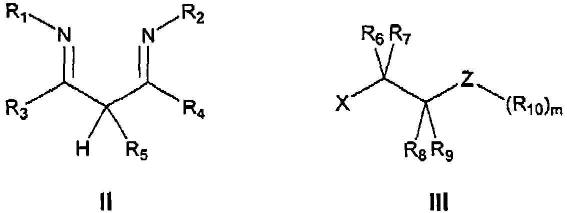 wherein R5 is hydrogen, followed by deprotonating the ligand formed, with a base.

R1 and R2 are the same or different and are unsubstituted or substituted alkyl groups, unsubstituted or substituted aryl groups, or unsubstituted or substituted cycloalkyl groups, more preferably, they are unsubstituted or substituted phenyl groups and if they are substituted, the substituents may be joined to form a closed structure. If the phenyls are substituted, the substituents preferably occupy 2 and 6 positions.

R3 and R4 are the same or different, hydrogen, unsubstituted or substituted alkyl groups, unsubstituted or substituted aryl groups, or unsubstituted or substituted cycloalkyl groups, more preferably, they are unsubstituted or substituted alkyl groups. Optionally R3 and R4 may also be linked together to form a cyclohexyl ring.

In another embodiment according to the present invention, R1 with R3 and/or R2 with R4 are linked together to form a ring.

Preferably, Z is selected from N, P, O or S.

R6, R7, R8, R9 and R10 are the same or different, hydrogen, unsubstituted or substituted alkyl groups, unsubstituted or substituted aryl groups, or unsubstituted or substituted cycloalkyl groups. R8 and R9 and/or R9 and R10 can be linked together to form a ring, for example a pyridine, a quinoline, an isoquinoline, a pyrrolyl, a furyl or a thiophenyl group.

Examples of formula III include 2-(bromomethyl)-5-nitrofuran, 2-(bromomethyl)-1,3-dioxalane, 2-(bromomethyl)tetrahydro-2H-pyran, 2-(bromomethyl)-5-trifluoromethyl)furan, 3(-bromomethyl)pyridazine, 2-bromomethylpyridine, 1-bromo-2-ethoxyethane, 2-bromoethylacetate, 1-bromo-2-(2-methoxyethoxy)ethane, [(2-bromoethoxy)methyl]benzene and 3-(bromomethyl)-2,4,10-trioxaadamantane, 2-bromo-N,N-dimethylaniline.

The invention also discloses a catalyst component of formula IV: 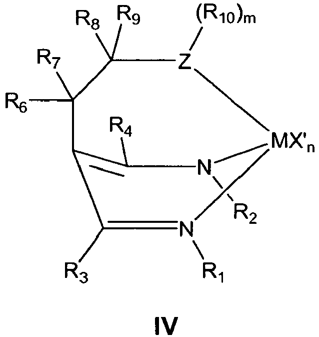 resulting from the complexation of deprotonated ligand I with the metallic salt MX'ₙ₊₁ in a solvent, wherein M is a metal Group 3 to 10 of the periodic Table, wherein X' is the same or different and can be a halogen, alcoholate, or substituted or unsubstituted hydrocarbyl, wherein n+1 is the valence of M and wherein said ligand I is deprotonated by a base and characterised in that pending arm 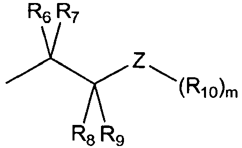 is folding to coordinate heteroatom Z to metal M.

The base used for deprotonating ligand I can be selected for example from butyl lithium, methyl lithium, sodium hydride. More preferably it is butyl lithium.

Preferably, M is Ti, Zr, Hf, V, Cr, Mn, Fe, Co, Ni, Pd or rare earths. More preferably, it is Cr or Ti.

Preferably, X' is halogen, more preferably it is chlorine.

The metal is complexed with the two nitrogen atoms of the starting beta-diimine and during the complexation reaction, the complex folds around in order to coordinate heteroatom Z to the metal to form a three dimensional scorpion-like structure.

The solvent may be selected from dichloromethane or tetrahydrofuran and the complexation reaction is carried out at room temperature.

The present invention also discloses an active catalyst system comprising the single site catalyst component of formula IV and an activating agent having an ionising action.

Suitable activating agents are well known in the art. The activating agent can be an aluminium alkyl represented by formula AlR⁺ₙX₃₋ₙ wherein R⁺ is an alkyl having from 1 to 20 carbon atoms and X is a halogen. The preferred aluminium alkyls are triisobutyl aluminium (TIBAL) or triethyl aluminium (TEAL).

Alternatively, it can be aluminoxane and comprise oligomeric linear and/or cyclic alkyl aluminoxanes represented by formula 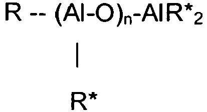 for oligomeric, linear aluminoxanes and by formula 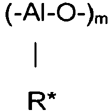 for oligomeric, cyclic aluminoxane,
wherein n is 1-40, preferably 1-20, m is 3-40, preferably 3-20 and R* is a C₁-C₈ alkyl group and preferably methyl or isobutyl.

Preferably, the activating agent is selected from methylaluminoxane (MAO) or tetraisobutyldialuminoxane (IBAO), more preferably, it is MAO.

The amount of activating agent is selected to give an Al/M ratio of from 100 to 3000, preferably of from 500 to 2000. The amount of activating agent depends upon its nature, the preferred Al/M ratio is of about 2000

Suitable boron-containing activating agents may comprise a triphenylcarbenium boronate such as tetrakis-pentafluorophenyl-borato-triphenylcarbenium as described in EP-A-0427696, or those of the general formula [L'-H] + [B Ar₁ Ar₂ X₃ X₄]- as described in EP-A-0277004 (page 6, line 30 to page 7, line 7).

The amount of boron-containing activating agent is selected to give a B/M ratio of from 0.5 to 5, preferably of about 1.

In another embodiment, according to the present invention, the single site catalyst component of formula IV may be deposited on a conventional support. Preferably, the conventional support is silica impregnated with MAO. Alternatively the support may also be an activating support such as fluorinated alumina silica.

The present invention further discloses a method for preparing an active catalyst system that comprises the steps of:
a) providing a beta-diimine ligand precursor of formula II;
b) reacting the beta-diimine ligand precursor of formula II with compound III to obtain a ligand;
c) deprotonating the ligand of step b) with a base;
d) complexing the deprotonated ligand of step c) with a metallic salt MX'ₙ₊₁;
e) retrieving a catalyst component of formula. IV;
f) activating the catalyst component of step e) with an activating agent having an ionising action;
g) optionally adding a cocatalyst;
h) retrieving an active oligomerisation or polymerisation catalyst system.

Alternatively, in step f) catalyst component IV is deposited on a support impregnated with an activating agent or on an activating support containing fluor.

The cocatalyst may be selected from triethylaluminium, triisobutylaluminum, tris-n-octylaluminium, tetraisobutyldialuminoxane or diethyl zinc.

The active catalyst system is used in the oligomerisation and in the polymerisation of ethylene and alpha-olefins.

The present invention discloses a method for the oligomerisation or the homo- or copolymerisation of ethylene and alpha-olefins that comprises the steps of:
a) injecting the active catalyst system into the reactor;
b) injecting the monomer and optional comonomer either before or after or simultaneously with step a);
c) maintaining under polymerisation conditions;
d) retrieving the oligomers and/or polymer.

The pressure in the reactor can vary from 0.5 to 50 bars, preferably from 5 to 25 bars.

The polymerisation temperature can range from 10 to 100°C, preferably from 50 to 85°C.

The preferred monomer and optional comonomer can be selected from ethylene, propylene or 1-hexene. Alternatively, the optional comonomer can be a polar functionalised alpha-olefin.

With a catalyst activated by MAO, the polymer formed is characterised by a melting point comprised between 130 and 135 °C as measured by Differential Scanning Calorimetry (DSC) method. They also have a high weight average molecular weight Mw. The molecular weight distribution is measured by the polydispersity index D defined as the ratio Mw/Mn of the weight average molecular weight Mw over the number average molecular weight Mn. Molecular weights are measured by Gel Permeation Chromatography (GPC)

### Examples.

### Preparation of catalyst components.

### Synthesis of ligand L1.

### Step 1.

The beta-diimine was synthesised according to the procedure published by Feldman and al. (Organometallics 1997, 16, p. 1514).

### Step 2.

760 mg (3 mmol) of 2-bromomethylpyridine.HBr and 436 mg (3.15 mmol) of potassium carbonate were degassed under vacuum for 1 hour. 10 mL of dry acetone were added and the mixture was stirred under argon for 6 hours at room temperature (about 25 °C). The solvent was removed and the 2-bromomethylpyridine was extracted with 3 x 10 mL of diethyl ether under inert atmosphere. The solvent was removed to afford a pink oil with quantitative yield.

### Step 3.

1.26 g (3 mmol) of beta-diimine were dissolved in 15 mL of dry THF under argon. The solution was cooled to a temperature of -20°C and 2 mL (3.15 mmol) of n-BuLi (1.6 M in hexane) were added dropwise. The colourless solution turned immediately bright yellow and was stirred at room temperature for 30 minutes. The solution was cooled to a temperature of -20°C and a solution of 2-bromomethylpyridine in 10 mL of dry THF was added by canula. The solution was allowed to warm to room temperature and was stirred overnight, before being heated at a temperature of 80°C for 6 hours under reflux. After that time, the solvent was evaporated to dryness. The residue was extracted with 10 mL of dichloromethane and filtered over neutral alumina. The solution was evaporated to afford a yellow oil that was purified by column chromatography (SiO₂, pentane:diethyl ether 95:5 to 80:20). 910 mg of the expected product were obtained as pale yellow oil containing isomers 1a and 1b, with a yield of 60 %.

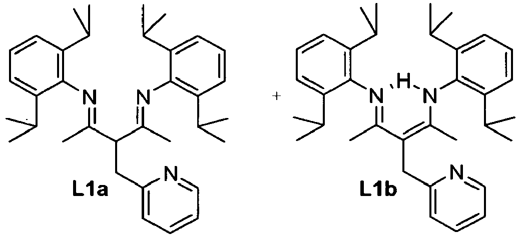

The isomers were characterised as follows:
C₃₅H₄₇N₃
M=509.77 g.mol⁻¹

¹H NMR (500 MHz, CDCl₃) results:
Isomer 1a: δ= 1.10 (m, 12H, CH₃ iPr), 1.21 (dd, 12H, J=6.8 Hz, CH₃ iPr), 1.92 (s, 6H, CH₃CN), 2.53 (sept, 2H, J=6.8 Hz, CH iPr), 2.60 (sept, 2H, J=6.8 Hz, CH iPr), 3.65 (d, 2H, J=7.5 Hz, CH₂), 4.70 (t, 1H, J=7.5 Hz, CH), 7.11 (m, 2H, CH para Ph), 7.17 (br s, 4H, CH meta Ph), 7.22 (m, 1H, H5 pyr), 7.38 (d, 1H, J=7.5 Hz, H3 pyr), 7.66 (td, 1H, J=7.5 Hz, J=1.8 Hz, H4 pyr), 8.66 (d, 1 H, J=5 Hz, H6 pyr).
Isomer **1b**: δ = 1.07 (m, 12H, CH₃ *i*Pr), 1.13 (d, 12H, J=6.9 Hz, CH₃ *i*Pr), 1.78 (s, 6H, CH₃CN), 3.20 (sept, 4H, J=6.9 Hz, CH *i*Pr), 4.06 (s, 2H, CH₂), 7.09 (m, 2H, CH para Ph), 7.13 (m, 4H, CH meta Ph), 7.18 (m, 1 H, H5 pyr), 7.33 (d, 1H, J=7.8 Hz, H3 pyr), 7.68 (m, 1 H, H4 pyr), 8.61 (d, 1 H, J=5 Hz, H6 pyr).

### Synthesis of ligand L2

Using the same procedure as that used to prepare ligand 1 (steps 1, 2 and 3), ligand L2 was obtained with a yield of 61 %. 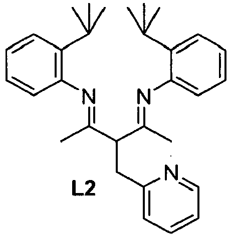 ¹H NMR (500 MHz, CDCl₃) : δ= 1.26 (s, 18H, CH₃ tBu), 2.00 (s, 6H, CH₃CN), 3.63 (d, 2H, J=7.5 Hz, CH₂), 4.44 (t, 1H, J=7.5 Hz, CH), 6.25 (d, 2H, J=7.5 Hz), 7.05 (m, 6H), 7.34 (t, 2H, J=7.5 Hz), 7.62 (t, 1H, J=7.5 Hz), 8.59 (d, 1H, J=5 H).

### Synthesis of ligand L3

Using the same procedure as that used to prepare ligand L1 (steps 1, 2 and 3), ligand L3 was obtained with a yield of 41%. 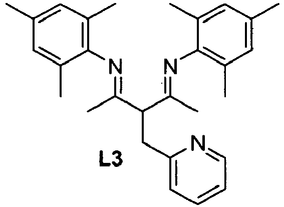 ¹H NMR (500 MHz, CDCl₃) : δ= 1.80 (s, 6H, CH₃), δ= 1.84 (s, 12H, CH₃), 2.25 (s, 6H, CH₃), 3.60 (d, 2H, J=7.5 Hz, CH₂), 4.53 (t, 1H, J=7.5 Hz, CH), 6.90 (s, 4H), 7.1-7.25 (m, 1 H), 7.30-7.80 (m, 3H), 8.58 (m, 1 H).

### Synthesis of ligand L4

Using the same procedure as that used to prepare ligand L1 (steps 1, 2 and 3) except that 2-bromomethylquinoline.HCl was used instead of 2-bromomethylpyridine.HBr, ligand L4 was obtained with a yield of 23%. 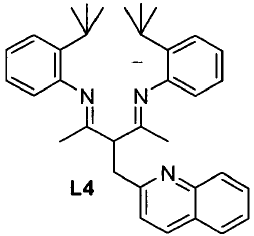 ¹H NMR (300 MHz, CDCl₃) : δ =1.22 (s, 18H, 2*tBu), 2.10 (s, 6H, 2*CH₃), 3.86 (d, 2H, J=7.2 Hz, CH₂), 4.68 (t, 1H, J=7.2 Hz, CH), 6.31 (d, 2H, J=7.5 Hz,2*CH Ph), 7.05 (m, 4H, 4*CH Ph), 7.35 (d, 2H, J=7.5 Hz, 2*CH Ph), 7.46 (d, 1 H, J=8.4 Hz, CH quin), 7.53 (t, 1 H, J=7.5 Hz, CH quin), 7.72 (t, 1 H, J=7.6 Hz, CH quin), 7.81 (d, 1H, J=8.1 Hz, CH quin), 809 (d, 2H, J=8.4 Hz, 2*CH quin).

### Synthesis of ligand L5

Using the same procedure as that used to prepare ligand L1 (steps 1, 2 and 3), except that 2-methoxy-5-nitrobenzyl bromide was used instead of 2-bromomethylpyridine.HBr, ligand L5 was obtained with a yield of 57%. 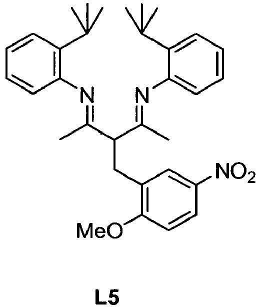 ¹H NMR (300 MHz, CDCl₃) : δ=1.29 (s, 18H, 2*tBu), 1.99 (s, 6H, 2*CH₃), 3.52 (d, 2H, J=7.5 Hz, CH₂); 4.00 (s, 3H, OCH₃), 4.17 (t, 1H, J=7.5 Hz, CH), 6.27 (dd, 2H), 7.05 (m, 6H), 7.37 (dd, 2H), 8.20 (s, 1H).

### Synthesis of ligand L6

Using the same procedure as that used to prepare ligand L1 (steps 1, 2 and 3), ligand L6 was obtained with a yield of 67%. 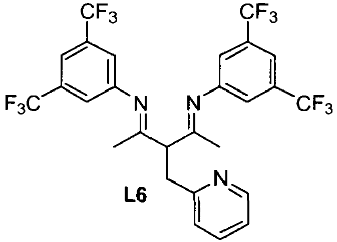 ¹H NMR (300 MHz, CDCl₃) : δ= 1.98 (s, 6H, CH₃), 3.52 (d, 2H, J=7.5 Hz, CH₂), 4.27 (t, 1H, J=7.5 Hz, CH), 7.03 (s, 4H), 7.21 (m, 1 H), 7.28 (d, 1 H, J=7.8 Hz), 7.68 (t, 1H, J=7.6 Hz), 8.61 (m, 1 H).

### Synthesis of ligand L7

Using the same procedure as that used to prepare ligand L1 (steps 1, 2 and 3), ligand L7 was obtained with a yield of 40%. 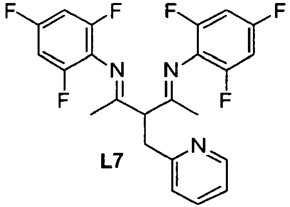 ¹H NMR (300 MHz, CDCl₃) δ= 1,94 (s, 6H, CH₃) ; 3,56 (d, 2H, d, J=7.5 Hz, CH₂) ; 4,42 (t, 1 H, t, J=7.5 Hz, CH) ; 6,68 (t, J_{HF}=8.3 Hz, 4H) ; 7,14 (m, 1 H), 7,26 (d, 1 H, J=7.5 Hz), 7,61 (t, 1 H, J=7.7 Hz), 8,56 (d, 1 H, J=3.9 Hz).

### Synthesis of ligand L8

Using the same procedure as that used to prepare ligand L1 (steps 1, 2 and 3), ligand L8 was obtained with a yield of 46%. 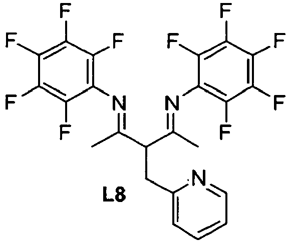 ¹H NMR (300 MHz, CDCl₃) : δ= 1.99 (s, 6H, CH₃), 3.56 (d, 2H, J=7.5 Hz, CH₂), 4.53 (t, 1 H, J=7.5 Hz, CH), 7.18 (m, 1 H), 7.26 (d, 1 H, J=7.8 Hz), 7.65 (t, 1 H, J=7.5 Hz), 8.56 (m, 1 H).

### Synthesis of ligand L9

Using the same procedure as that used to prepare ligand L1 (steps 1, 2 and 3), ligand L9 was obtained with a yield of 69%. 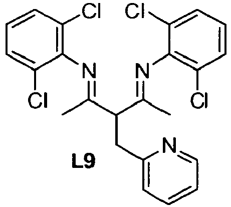 ¹H NMR (300 MHz, CDCl₃) : δ= 2.00 (s, 6H, CH₃), 3.65 (d, 2H, J=7.8 Hz, CH₂), 4.67 (t, 1 H, J=7.8 Hz, CH), 6.92 (t, 2H, J=8.1 Hz), 7.14 (m, 1 H), 7.26 (m, 4H), 7.28 (m, 1 H), 7.61 (t, 1 H, J=7.6 Hz), 8.60 (m, 1 H).

### Synthesis of ligand L10

Using the same procedure as that used to prepare ligand L1 (steps 1, 2 and 3), ligand L10 was obtained with a yield of 39%. 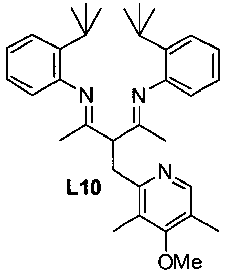 ¹H NMR (300 MHz, CDCl₃) : δ = 1,25 (s, 18H, tBu), 2,05 (s, 6H, CH₃), 2,27 (s, 3H, CH₃ pyr), 2,31 (s, 3H, CH₃ pyr), 3,56 (d, 2H, J=7.2 Hz, CH₂), 3,75 (s, 3H, OCH₃), 4,58 (t, 1 H, J=7.2 Hz, CH), 6,33 (d, J=7.6 Hz, 2H), 7,01 (t, J=7.5 Hz, 2H), 7,10 (t, J=7.4 Hz, 2H), 7,35 (d, J=7.8 Hz, 2H), 8,21 (s, 1H).

### Synthesis of ligand L11

Using the same procedure as that used to prepare ligand L1 (steps 1, 2 and 3), ligand L11 was obtained with a yield of 76%. 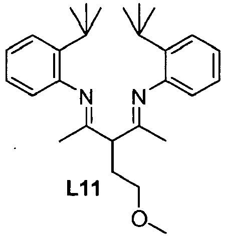 ¹H NMR (300 MHz, CDCl₃) : δ= 1,37 (s, 18H, tBu), 1,97 (s, 6H, CH₃), 2,41 (q, 2H, J=6.6 Hz, CH₂), 3,42 (s, 3H, OCH₃), 3,62 (t, 2H, J=6.4 Hz, CH₂O), 3,77 (t, 1 H, J=7.1 Hz, CH), 6,41 (d, 2H, J=7.6 Hz), 7,04 (t, 2H, J=7.5 Hz), 7,14 (t, 2H, J=7.5 Hz), 7,38 (d, 2H, J=7.8 Hz).

### Method 1:Preparation of Cr(III) complexes

0.5 mmol of the appropriate ligand were dissolved in 5 mL of tetrahydrofuran. 0.5 mmol of n-butyl lithium were added dropwise at a temperature of -15°C. The solution was allowed to warm to room temperature and was stirred for 30 minutes. The solution was added dropwise to a solution of 0.5 mmol of chromium (III) chloride tetrahydrofuran complex dissolved in 5 mL of dry tetrahydrofuran. The mixture was stirred under argon overnight at room temperature. The solution was concentrated to approximately 2 mL and 10 mL of pentane were added. The solid was filtered off, washed twice with 5 mL of pentane and dried under vacuum.

### Method 2: Preparation of Ti(IV) complexes

0.35 mmol of the appropriate ligand were dissolved in 5 mL of tetrahydrofuran. 0.35 mmol of n-butyl lithium were added dropwise at a temperature of -78°C. The solution was allowed to warm to room temperature and was stirred for 30 minutes. The solution was added dropwise to a solution of 0.35 mmol of titanium (IV) tetrachloride dissolved in 5 mL of dry tetrahydrofuran cooled at a temperature of -78°C. The mixture was stirred under argon overnight at room temperature. The solution was concentrated to approximately 2 mL and 10 mL of pentane were added. The solid was filtered off, washed twice with 10 mL of pentane and dried under vacuum.

### Method 3: Preparation of Zr(IV) complexes

0.35 mmol of the appropriate ligand were dissolved in 5 mL of tetrahydrofuran. 0.35 mmol of n-butyl lithium were added dropwise at a temperature of -78°C. The solution was allowed to warm to room temperature and was stirred for 30 minutes. The solution was added dropwise to a solution of 0.35 mmol of zirconium (IV) tetrachloride dissolved in 5 mL of dry tetrahydrofuran cooled at a temperature of - 78°C. The mixture was stirred under argon overnight at 70°C. The solution was concentrated to approximately 2 mL and 10 mL of pentane were added. The solid was filtered off, washed twice with 10 mL of pentane and dried under vacuum.

### Method 4: Preparation of V(III) complexes

0.35 mmol of the appropriate ligand were dissolved in 5 mL of tetrahydrofuran. 0.35 mmol of n-butyl lithium were added dropwise at a temperature of -78°C. The solution was allowed to warm to room temperature and was stirred for 30 minutes. The solution was added dropwise to a solution of 0.35 mmol of vanadium (III) trichloride tetrahydrofuran complex dissolved in 5 mL of dry tetrahydrofuran cooled at a temperature of -78°C. The mixture was stirred under argon overnight at room temperature. The solution was concentrated to approximately 2 mL and 10 mL of pentane were added. The solid was filtered off, washed twice with 10 mL of pentane and dried under vacuum.

### Method 5: Preparation of Fe(III) complexes

0.35 mmol of the appropriate ligand were dissolved in 5 mL of tetrahydrofuran. 0.35 mmol of n-butyl lithium were added dropwise at a temperature of -20°C. The solution was allowed to warm to room temperature and was stirred for 30 minutes. The solution was added dropwise to a solution of 0.35 mmol of iron (III) trichloride dissolved in 5 mL of dry tetrahydrofuran cooled at a temperature of -20°C. The mixture was stirred under argon overnight at room temperature. The solution was concentrated to approximately 2 mL and 10 mL of pentane were added. The solid was filtered off, washed twice with 10 mL of pentane and dried under vacuum.

Using method 1, Cr(III) complex 1 was obtained as a pale grey solid with a yield of 74%.

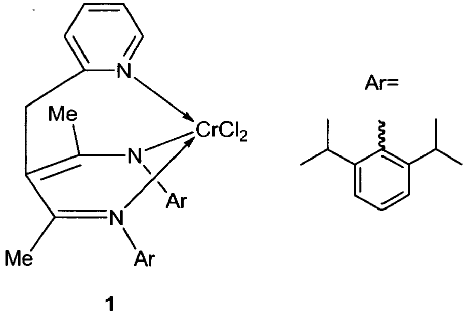

Using method 1, Cr(III) complex 2 was obtained as a pale green solid with a yield of 75%.

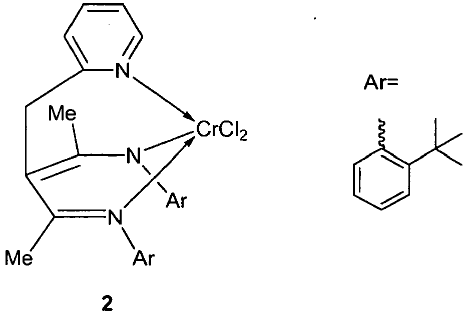

Using method 1, Cr(III) complex 3 was obtained as a pale green solid with a yield of 48%.

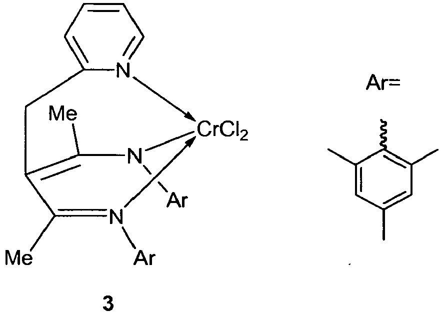

Using method 2, Ti(IV) complex 4 was obtained as a brown solid with a yield of 94%.

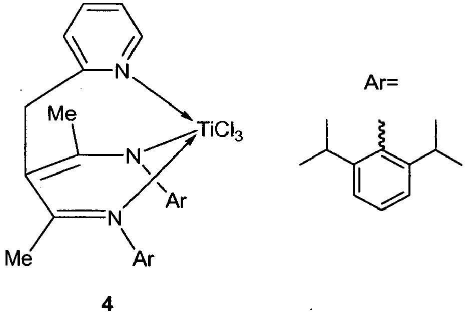

Using method 2, Ti(IV) complex 5 was obtained as a brown solid with a yield of 94%.

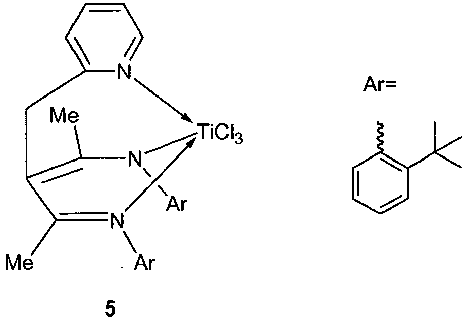

Using method 2, Ti(IV) complex 6 was obtained as a brown solid with a yield of 75%.

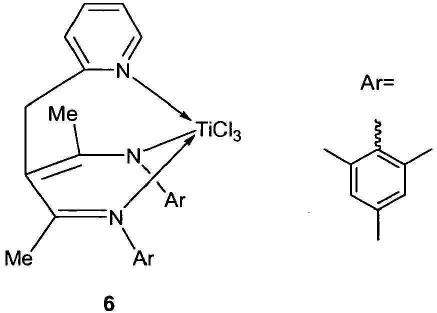

Using method 1, Cr(III) complex 7 was obtained.

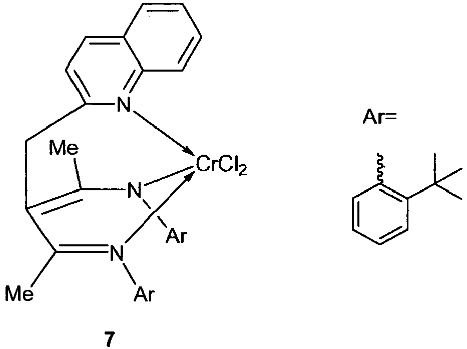

Using method 2, Ti(IV) complex 8 was obtained as a orange solid with a yield of 56%.

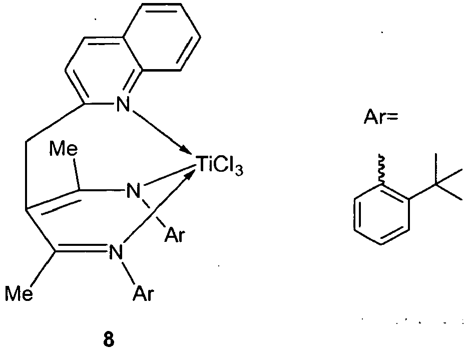

Using method 3, Zr(IV) complex 9 was obtained.

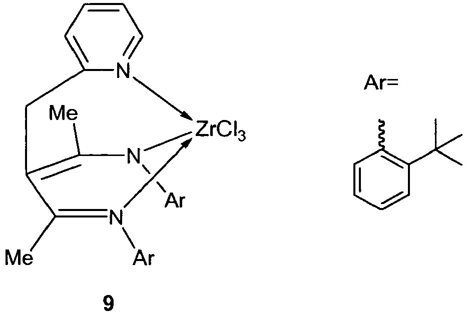

Using method 4, V(III) complex 10 was obtained as a green solid with a yield of 76%.

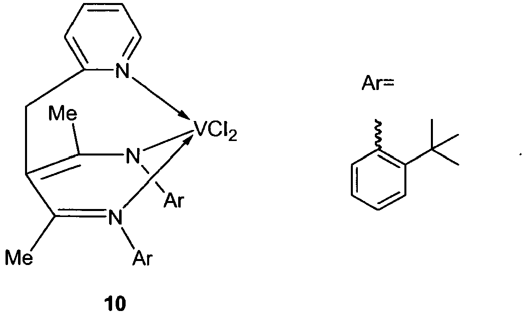

Using method 5, Fe(III) complex 11 was obtained as a brown solid with a yield of 63%.

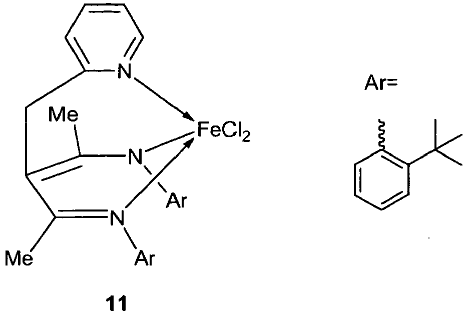

Using method 1, Cr(III) complex 12 was obtained as a green solid with a yield of 34%.

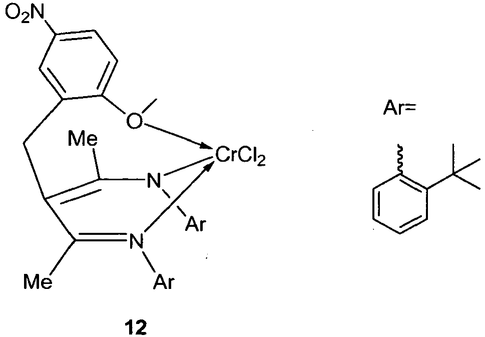

Using method 2, Ti(IV) complex 13 was obtained as a dark brown solid with a yield of 50%.

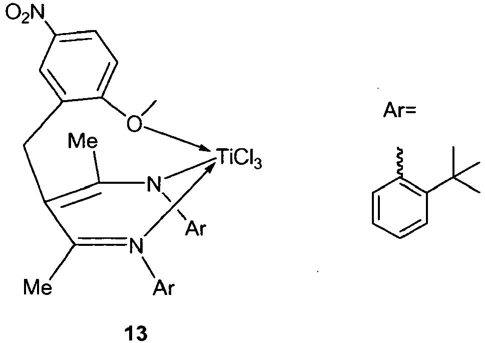

Using method 2, Ti(IV) complex 14 was obtained as a clear brown solid with a yield of 70%.

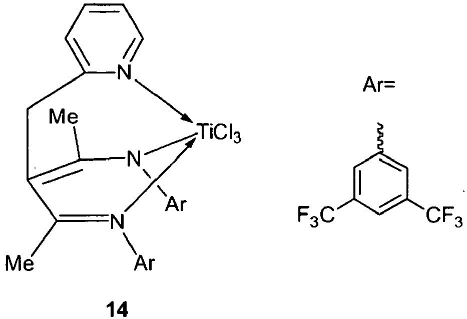

Using method 2, Ti(IV) complex 15 was obtained as a black solid with a yield of 44%.

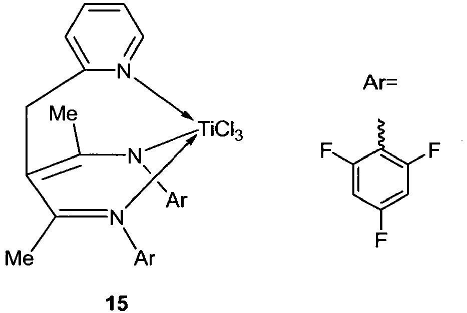

Using method 2, Ti(IV) complex 16 was obtained as a red solid with a yield of 19%.

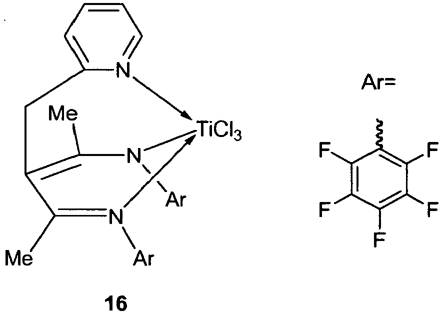

Using method 2, Ti(IV) complex 17 was obtained as a yellow solid with a yield of 81%.

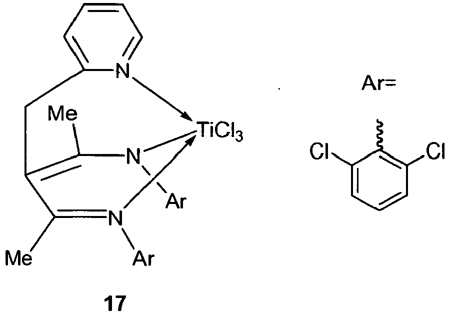

Using method 2, Ti(IV) complex 18 was obtained as an orange solid with a yield of 34%.

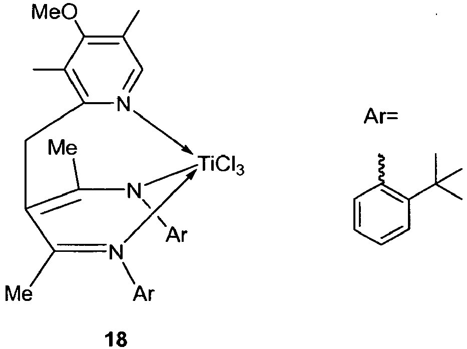

Using method 2, Ti(IV) complex 19 was obtained as a clear brown-orange solid with a yield of 52%.

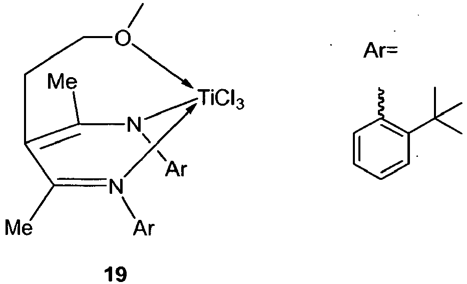

Using method 3, Zr(IV) complex 20 was obtained as a yellow solid with a yield of 63%.

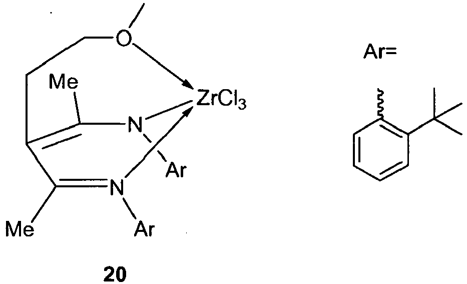

### High pressure polymerisation of ethylene.

Ethylene polymerisation reactions were performed in a 20 mL stainless steel autoclave containing a glass insert, fitted with mechanical stirring, external thermocouple and pressure gauge and controlled by computer. In a typical reaction run, 4 mL of dry solvent (toluene or n-heptane) were introduced into the reactor and the temperature and ethylene pressure were raised to the desired values. Ethylene was fed continuously. In an argon-filled glove box, about 5 µmol of the appropriate catalyst were weighted. It was activated with methylaluminoxane (MAO 30 % wt in toluene) in an amount appropriate to obtain a ratio [Al]:[M] of 2000 and it was diluted with toluene to a final volume of 2 mL. 200 µL of the solution of activated catalyst were placed inside the reactor. The injection loop was rinsed with 800 µL of solvent. After 1 hour or after an ethylene consumption of 12 mmol, the reaction was quenched with isopropanol and an aliquot was analysed by gas chromatography. The gas chromatographic analysis of the reaction products were performed on a Trace GC apparatus with a Petrocol capillary column (methyl silicone, 100 m long, i.d. 0.25 mm and film thickness of 0.5 µm) working at a temperature of 35°C for 15 min and then heated to a temperature of 250 °C at a heating rate of 5°C/min. The remaining reaction mixture was quenched with MeOH / HCl and the polymer was filtered, washed with methanol and dried at a temperature of 50°C, under vacuum, for a period of time of 24 hours.

The results are displayed in Table I and in Table II.

**TABLE I. Results with the Cr(III) complexes**

| **Run** | **Complex** | **P (bar)** | **T (°C)** | **m PE (mg)** | **Activity (kg/mol/h)** | **DSC** | |
|---|---|---|---|---|---|---|---|
| | | | | | | **Tm(°C)** | **ΔH(J.g⁻¹)** |
| 1 | **1** | 15 | 50 | 47 | 93 | 132 | 165.5 |
| 2 | **2** | 15 | 50 | 45 | 86 | 132 | 178.5 |
| 3 | **3** | 15 | 50 | 40 | 78 | 132 | 177.8 |
| 4 | **7** | 15 | 50 | 36 | 70 | 130 | 170.9 |
| 5 | **12** | 15 | 50 | 41 | 81 | 131 | 170.6 |
| 6 | **1** | 19 | 80 | 27 | 53 | 131 | 202.5 |
| 7 | **2** | 19 | 80 | 26 | 52 | 131 | 209.1 |
| 8 | **3** | 19 | 80 | 32 | 63 | 130 | 183.5 |

All reactions were performed with 0.5 µmol of catalyst dissolved in 5 mL of n-heptane during 1 hour.
MAO was added to give a [Al]:[Cr] ratio of 2000.
Activities are expressed in kg of polyethylene per mol Cr per hour.

**TABLE II. Results with the Ti(IV) complexes**

| **Run** | **Complex** | **P (bar)** | **T (°C)** | **Time (min)** | **m PE(mg)** | **Activity (kg/mol/h)** | **DSC** | |
|---|---|---|---|---|---|---|---|---|
| | | | | | | | **Tm (°C)** | **ΔH (J.g⁻¹)** |
| 9 | 4 | 15 | 50 | 60 | 552 | 1111 | 135 | 133,3 |
| 10 | 5 | 15 | 50 | 60 | 630 | 1198 | 135 | 122,1 |
| 11 | 6 | 15 | 50 | 60 | 583 | 1027 | 133 | 138,6 |
| 12 | 4 | 19 | 80 | 51 | 812 | 1909 | 134 | 136,9 |
| 13 | 5 | 19 | 80 | 48 | 862 | 2094 | 135 | 145,7 |
| 14 | 6 | 19 | 80 | 52 | 955 | 2187 | 136 | 150,4 |
| 15 | 8 | 19 | 80 | 60 | 257 | 512 | 134 | 120.0 |
| 16 | 13 | 19 | 80 | 60 | 274 | 549 | 134 | 125.0 |
| 17 | 14 | 19 | 80 | 60 | 408 | 1646 | 136 | 108.2 |
| 18 | 15 | 19 | 80 | 60 | 301 | 1209 | 139 | 119.1 |
| 19 | 16 | 19 | 80 | 60 | 705 | 1399 | 132 | 98.7 |
| 20 | 17 | 19 | 80 | 60 | 387 | 765 | 133 | 129.7 |
| 21 | 18 | 19 | 80 | 60 | 260 | 514 | 133 | 96.6 |
| 22 | 19 | 19 | 80 | 60 | 383 | 749 | 138 | 109.2 |

All reactions were performed with 0.5 µmol of catalyst dissolved in 5 mL of n-heptane during 1 hour.
MAO was added to give a [Al]:[Ti] ratio of 2000.
Activities are expressed in kg of polyethylene per mol Ti per hour.

**TABLE III. Results with the Zr(IV) complex**

| **Run** | **Complex** | **P(bar)** | **T(°C)** | **mPE (mg)** | **Activity (kg/mol/h)** | **DSC** | |
|---|---|---|---|---|---|---|---|
| | | | | | | **Tm (°C)** | **ΔH(J.g⁻¹)** |
| 23 | **9** | 15 | 50 | 395 | 778 | 133 | 130.1 |
| 24 | **9** | 19 | 80 | 473 | 932 | 134 | 148.9 |
| 25 | **20** | 19 | 80 | 249 | 480 | 138 | 137.7 |

All reactions were performed with 0.5 µmol of catalyst dissolved in 5 mL of n-heptane during 1 hour.
MAO was added to give a [Al]:[Zr] ratio of 2000.
Activities are expressed in kg of polyethylene per mol Zr per hour.

**TABLE IV. Results with the V(III) complex**

| **Run** | **Complex P** | **(bar)** | **T (°C)** | **m PE (mg)** | **Activity (kg/mol/h)** | **DSC** | |
|---|---|---|---|---|---|---|---|
| | | | | | | **Tm(°C)** | **ΔH (J.g⁻¹)** |
| 25 | **10** | 15 | 50 | 52 | 103 | 133 | 139.1 |
| 27 | **10** | 19 | 80 | 63 | 125 | 133 | 146.8 |

All reactions were performed with 0.5 µmol of catalyst dissolved in 5 mL of n-heptane during 1 hour.
MAO was added to give a [Al]:[V] ratio of 2000.
Activities are expressed in kg of polyethylene per mol V per hour.

### Polymerisation of ethylene with a supported catalyst

Complex 5 was deposited on a MAO impregnated silica (Sylopol 952X1836), with 2 wt % of complex based on the total weight of the obtained supported catalyst. This supported catalyst was used for the polymerisation of ethylene.

Ethylene polymerisation reactions were carried out in a 130 ml stainless steel autoclave equipped with mechanical stirring and a stainless steel injection cylinder. In a typical reaction run, the reactor was first dried under nitrogen flow at 100°C during 10 min. Then it was cooled down to the reaction temperature (85°C) and 35 ml of isobutane were introduced into the reactor with a syringe pump. The pressure was adjusted to the desired value (23.8 bar) with ethylene. In an argon-filled glove box, 301 mg of the supported catalyst, 0.6 ml of TiBAl and 0.5 ml of n-heptane were placed into the injection cylinder. The valve was closed and the cylinder was connected to the reactor under nitrogen flow. The active catalyst mixture was then introduced into the reactor with 40 ml of isobutane. After 1 hour at 750 rpm, the reactor was cooled down to room temperature and slowly depressurised. The polymer was recovered and characterised by DSC. The polymerisation results are displayed in Table V.

**TABLE V: Polymerisation results with supported Ti(IV) complex 5**

| **Run** | **Supported complex** | **PE g** | **Activity* g/g/h** | **Tm (°C)** |
|---|---|---|---|---|
| 28 | 5 | 3.2 | 10.6 | 133 |
| 29 | 5 | 2.9 | 9.7 | 133 |

| | | | | |
|---|---|---|---|---|
| * Activity is expressed as g of polyethylene per g of supported catalyst per hour. | | | | |

Due to the high molecular weight of the obtained polymers, GPC and ¹³C NMR analysis were not possible, even in TCB at 135°C.

## Claims

1. A deprotonated ligand of general formula I, 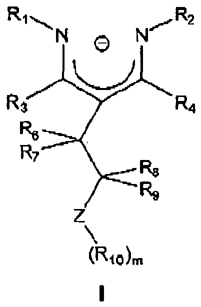 wherein R1 and R2 are the same or different and are unsubstituted or substituted alkyl groups, unsubstituted or substituted aryl groups, or unsubstituted or substituted cycloalkyl groups
wherein R3 and R4 are the same or different, and are hydrogen, unsubstituted or substituted alkyl groups, unsubstituted or substituted aryl groups, or unsubstituted or substituted cycloalkyl groups,
wherein R6, R7, R8, R9 and R10 are the same or different, and are hydrogen, unsubstituted or substituted alkyl groups, unsubstituted or substituted aryl groups, or unsubstituted or substituted cycloalkyl groups,
and wherein two or more of said R's can be linked together to form one or more rings R₈ and R₉ and/or R₉ and R₁₀ can be linked together to form a ring, with the restriction that R1 and R3, R2 and R4, and R9 and R10, cannot all be simultaneously oxazolin, and
wherein Z is selected from groups 15 or 16 of the Periodic Table and m is the valence of Z minus one.

2. The deprotonated ligand of claim 2 wherein R1 and R2 are the same and are unsubstituted or substituted phenyl groups.

3. The deprotonated ligand of claim 4 wherein R3 and R4 are unsubstituted or substituted alkyl groups or are linked together to form a cyclohexyl ring.

4. The deprotonated ligand of any one of the preceding claims wherein R1 with R3 and/or R2 with R4 are linked together to form a ring.

5. The deprotonated ligand of any one of the preceding claims wherein Z is selected from N, P, O or S.

6. A catalyst component of formula IV: 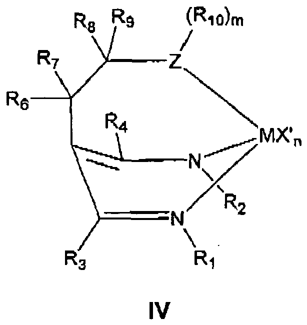 resulting from the complexation of deprotonated ligand I of any one of claims 1 to 5 with the metallic salt MX'ₙ₊₁ in a solvent, wherein M is a metal Group 3 to 10 of the periodic Table, X' is the same or different and is halogen, alcoholate, or substituted or unsubstituted hydrocarbyl and wherein n+1 is the valence of M and said metallic complex being **characterised in that** pending arm 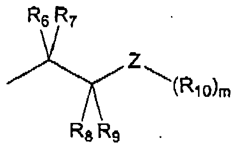 is folding to coordinate heteroatom Z to metal M.

7. The metallic complex of claim 6 wherein M is Ti, Zr, Hf, V, Cr, Mn, Fe, Co, Ni, Pd.

8. An active catalyst system comprising the metallic complex of claim 6 or claim 7 and an activating agent having an ionising action.

9. A method for preparing an active catalysts system that comprises the steps of:
a) providing a beta-diimine ligand precursor of formula II;
b) reacting the beta-diimine ligand precursor of formula II with compound III to obtain a ligand;
c) deprotonating the ligand of step b) by subtracting the R5 hydrogen with a base;
d) complexing the deprotonated ligand of step c) with a metallic salt MX'ₙ₊₁;
e) retrieving a catalyst component of formula IV;
f) activating the catalyst component of step e) with an activating agent having an ionising action;
g) optionally adding a cocatalyst;
h) retrieving an active oligomerisation or polymerisation catalyst system.

10. A method for the oligomerisation or for the homo- or co-polymerisation of ethylene and alpha-olefins that comprises the steps of:
a) injecting the active catalyst system of claim 9 into the reactor;
b) injecting the monomer and optional comonomer either before or after or simultaneously with step a);
c) maintaining under polymerisation conditions;
d) retrieving the oligomers and/or polymer.

11. The method of claim 10 wherein the monomer and optional comonomer are selected from ethylene, propylene and 1-hexene.

## Patentansprüche

1. Deprotonierter Ligand der Formel I 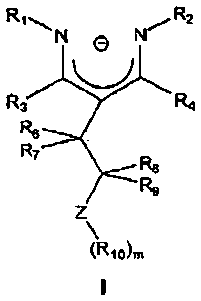 wobei R1 und R2 gleich oder unterschiedlich und unsubstituierte oder substituierte Alkylgruppen, unsubstituierte oder substituierte Arylgruppen oder unsubstituierte oder substituierte Cycloalkylgruppen sind,
wobei R3 und R4 gleich oder unterschiedlich und Wasserstoff, unsubstituierte oder substituierte Alkylgruppen, unsubstituierte oder substituierte Arylgruppen oder unsubstituierte oder substituierte Cycloalkylgruppen sind,
wobei R6, R7, R8, R9 und R10 gleich oder unterschiedlich und Wasserstoff, substituierte Alkylgruppen, unsubstituierte oder substituierte Arylgruppen oder unsubstituierte oder substituierte Cycloalkylgruppen sind, und
wobei zwei oder mehrere der Rs miteinander verknüpft sein können, um einen oder mehrere Ringe zu bilden, wobei R8 und R9 und/oder R9 und R10 miteinander verknüpft sein können, um einen Ring zu bilden, mit der Einschränkung, dass R1 und R3, R2 und R4 und R9 und R10 nicht alle gleichzeitig Oxazolin sein können, und
wobei Z aus den Gruppen 15 und 16 des Periodensystems ausgewählt ist, und m die Valenz von Z minus 1 ist.

2. Deprotonierter Ligand nach Anspruch 2, wobei R1 und R2 gleich und unsubstituierte oder substituierte Phenylgruppen sind.

3. Deprotonierter Ligand nach Anspruch 4, wobei R3 und R4 unsubstituierte oder substituierte Alkylgruppen oder miteinander verknüpft sind, um einen Cyclohexylring zu bilden.

4. Deprotonierter Ligand nach einem der vorstehenden Ansprüche, wobei R1 mit R3 und/oder R2 mit R4 miteinander verknüpft sind, um einen Ring zu bilden.

5. Deprotonierter Ligand nach einem der vorstehenden Ansprüche, wobei Z aus N, P, O oder S ausgewählt ist.

6. Katalysatorkomponente der Formel IV: 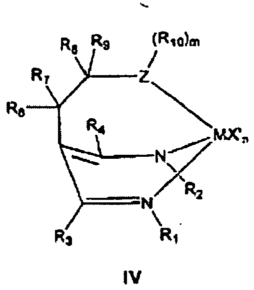 entstehend aus der Komplexierung des deprotonierten Liganden I nach einem der Ansprüche 1 bis 5 mit dem Metallsalz MX'ₙ₊₁ in einem Lösungsmittel, wobei M eine Metallgruppe 3 bis 10 des Periodensystems ist, X' gleich oder unterschiedlich und Halogen, Alkoholat oder substituiertes oder unsubstituiertes Hydrocarbyl ist, und wobei n+1 die Valenz M ist, und wobei der Metallkomplex **dadurch gekennzeichnet ist, dass** sich der anhängige Arm faltet, um Heteroatom Z in Bezug auf Metall M zu koordinieren.

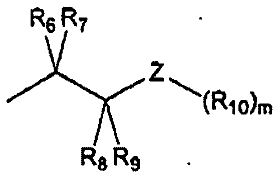

7. Metallkomplex nach Anspruch 6, wobei M Ti, Zr, Hf, V, Cr, Mn, Fe, Co, Ni, Pd ist.

8. Aktives Katalysatorsystem, umfassend den Metallkomplex von Anspruch 6 oder 7 und ein Aktivierungsmittel mit ionisierender Wirkung.

9. Verfahren zum Herstellen eines aktiven Katalysatorsystems, das die folgenden Schritte umfasst:
a) Bereitstellen eines beta-Diiminligandenpräkursors der Formel II;
b) Reagieren des beta-Diiminligandenpräkursors der Formel II mit Verbindung III, um einen Liganden zu erhalten;
c) Deprotonieren des Liganden von Schritt b) durch Subtrahieren des R5-Wasserstoffs mit einer Base;
d) Komplexieren des deprotonierten Liganden von Schritt c) mit einem Metallsalz MX'ₙ₊₁;
e) Gewinnen einer Katalysatorkomponente der Formel IV;
f) Aktivieren der Katalysatorkomponente von Schritt e) mit einem Aktivierungsmittel, das eine ionisierende Wirkung aufweist;
g) optional Hinzufügen eines Cokatalysators;
h) Gewinnen eines aktiven Oligomerisations- oder Polymerisationskatalysatorsystems.

10. Verfahren zur Oligomeration oder zur Homo- oder Copolymerisation von Ethylen und alpha-Olefinen, wobei das Verfahren die folgenden Schritte umfasst;
a) Injizieren des aktiven Katalysatorsystems nach Anspruch 9 in den Reaktor;
b) Injizieren des Monomers und optionalen Comonomers entweder bevor, nach oder gleichzeitig mit Schritt a);
c) Halten unter Polymerisationsbedingungen;
d) Gewinnen des Oligomers and/oder Polymers.

11. Verfahren nach Anspruch 10, wobei das Monomer und das optionale Comonomer aus Ethylen, Propylen und 1-Hexen ausgewählt sind.

## Revendications

1. Ligand déprotoné de formule générale I 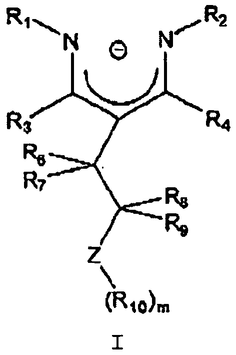 dans laquelle R1 et R2 sont identiques ou différents et sont des groupes alkyle substitués ou non substitués, des groupes aryle substitués ou non substitués, ou des groupes cycloalkyle substitués ou non substitués,
dans laquelle R3 et R4 sont identiques ou différents et sont des hydrogènes, des groupes alkyle substitués ou non substitués, des groupes aryle substitués ou non substitués, ou des groupes cycloalkyle substitués ou non substitués,
dans laquelle R6, R7, R8, R9 et R10 sont identiques ou différents et sont des hydrogènes, des groupes alkyle substitués ou non substitués, des groupes aryle substitués ou non substitués, ou des groupes cycloalkyle substitués ou non substitués,
et dans laquelle deux ou plus desdits R peuvent être liés ensemble pour former un ou plusieurs cycles, R8 et R9 et/ou R9 et R10 peuvent être liés ensemble pour former un cycle, avec la limitation suivante : R1 et R3, R2 et R4, et R9 et 10, ne peuvent pas tous être simultanément l'oxazoline, et
dans laquelle Z est choisi parmi les Groupes 15 et 16 du Tableau Périodique et m est la valence de Z moins un.

2. Ligand déprotoné selon la revendication 2, dans lequel R1 et R2 sont identiques et sont des groupes phényle substitués ou non substitués.

3. Ligand déprotoné selon la revendication 4, dans lequel R3 et R4 sont des groupes alkyle substitués ou non substitués ou bien sont liés ensemble pour former un cycle cyclohexyle.

4. Ligand déprotoné selon l'une quelconque des revendications précédentes, dans lequel R1 avec R3 et/ou R2 avec R4 sont liés ensemble pour former un cycle.

5. Ligand déprotoné selon l'une quelconque des revendications précédentes, dans lequel Z est choisi parmi N, P, O et S.

6. Composant de catalyseur de formule IV : 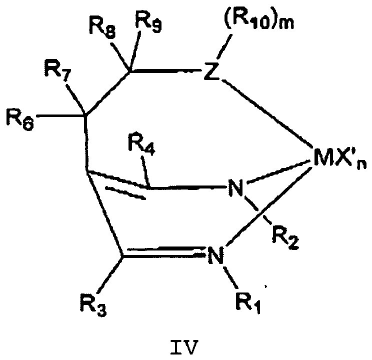 résultant de la complexation du ligand déprotoné I de l'une quelconque des revendications 1 à 5 avec un sel métallique MX'ₙ₊₁ dans un solvant, où M est un métal des Groupes 3 à 10 du Tableau Périodique, les X' sont identiques ou différents et sont un halogène, un alkylate ou un hydrocarbyle substitué ou non substitué, et où n+1 est la valence de M, ledit complexe métallique étant **caractérisé en ce que** le bras pendant 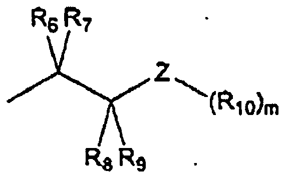 est replié pour coordonner l'hétéroatome Z au métal M.

7. Complexe métallique selon la revendication 6, dans lequel M est Ti, Zr, Hf, V, Cr, Mn, Fe, Co, Ni, Pd.

8. Système catalyseur actif comprenant le complexe métallique de la revendication 6 ou 7 et un agent d'activation ayant une action ionisante.

9. Procédé pour préparer un système catalyseur actif qui comprend les étapes consistant à :
a) disposer d'un précurseur de ligand bêta-diimine de formule II ;
b) faire réagir le précurseur de ligand bêta-diimine de formule II avec le composé III pour obtenir un ligand ;
c) déprotoner le ligand de l'étape b) en soustrayant l'hydrogène R5 avec une base ;
d) complexer le ligand déprotoné de l'étape c) avec un sel métallique MX'ₙ₊₁ ;
e) récupérer le composant de catalyseur de formule IV ;
f) activer le composant de catalyseur de l'étape e) avec un agent d'activation ayant une action ionisante ;
g) éventuellement ajouter un cocatalyseur ;
h) récupérer un système catalyseur actif d'oligomérisation ou de polymérisation.

10. Procédé pour l'oligomérisation ou pour l'homo- ou co-polymérisation d'éthylène et d'alpha-oléfines, qui comprend les étapes consistant à :
a) injecter le système catalyseur actif de la revendication 9 dans un réacteur ;
b) injecter le monomère et le comonomère éventuel, soit avant soit après soit en même temps que l'étape a) ;
c) maintenir dans des conditions de polymérisation ;
d) récupérer les oligomères et/ou polymères.

11. Procédé selon la revendication 10, dans lequel le monomère et le comonomère éventuel sont choisis parmi l'éthylène, le propylène et le 1-hexène.
